(19) Europäisches Patentamt
European Patent Office
Office européen des brevets

(11) Publication number: 0 484 596 A1

(12) EUROPEAN PATENT APPLICATION

(21) Application number: 90202936.2

(51) Int. Cl.⁵: A61K 31/555, C07F 7/22

(22) Date of filing: 06.11.90

(43) Date of publication of application:
13.05.92 Bulletin 92/20

(84) Designated Contracting States:
AT BE CH DE DK ES FR GB GR IT LI LU NL SE

(71) Applicant: PHARMACHEMIE B.V.
Swensweg 5
NL-2031 GA Haarlem(NL)

(72) Inventor: Gielen, Marcel
Bos van Houthulstlaan 6
B-1970 Wezembeek-Oppem(BE)
Inventor: Pan, Huade
Dienst AOSC, kamer 8G510, Pleinlaan 2
B-1050 Brussel(BE)
Inventor: Willem, Rudolph
H. Consciencestraat 7
B-1800 Vilvoorde(BE)
Inventor: De Vos, Dirk
Hofbrouckerlaan 36
NL-2341 LP Oegstgeest(NL)

(74) Representative: Ellowicz, Leo, Drs. et al
Octrooibureau Polak & Charlouis Laan
Copes van Cattenburch 80
NL-2585 GD Den Haag(NL)

(54) **Organotin compounds and anti-tumor compositions containing them.**

(57) The invention relates to anti-tumor compositions containing as an active ingredient one or more compounds of the formulae:

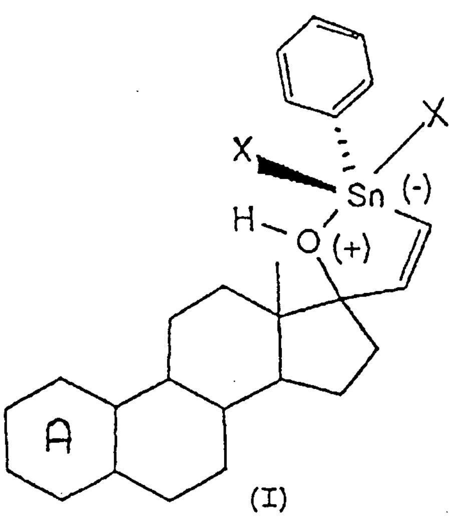
(I)

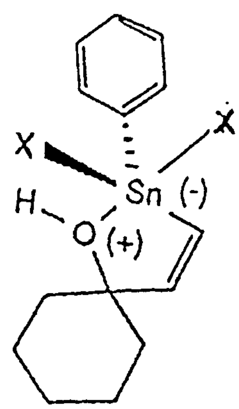
(II)

EP 0 484 596 A1

in particular compounds having the formulae (1), (2) and (3)

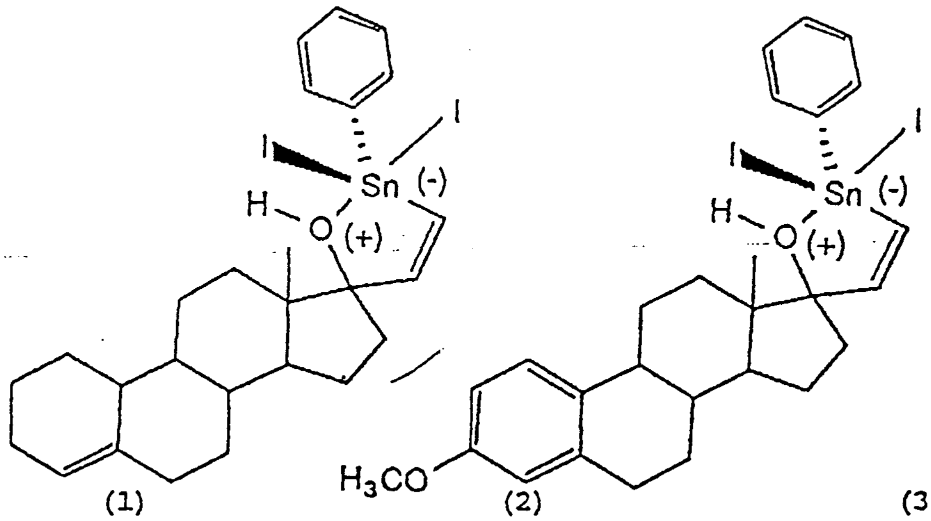

(1) (2)

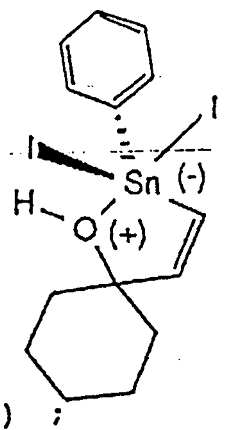

(3) ;

and to anti-tumor compositions containing a compound having the formula (6)

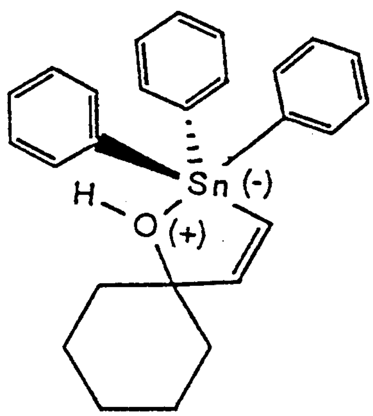

(6)

and to novel compounds, in particular of the formulae (2), (3) and 40 (6).

Numerous organo tin compounds having anti-tumour properties are known from literature, e.g. compounds of which the organic moiety is a steroid which is bound by one or two oxygen atoms to tin. For example, S.V. Kanakkanatt, "Synthesis of Tin Steroids and the Relation between Structure and Anticancer Activity", Chapter 15, pages 189-195 and N.F. Cardarelli, "Suppression of Mammary Adenocarcinoma in A/KI Mice through the Oral Administration of Tin Steroids", Chapter 17, pages 199-209, disclose triphenyltin cholate, prepared from cholic acid and triphenyltin hydroxide, and its anti-cancer activity.

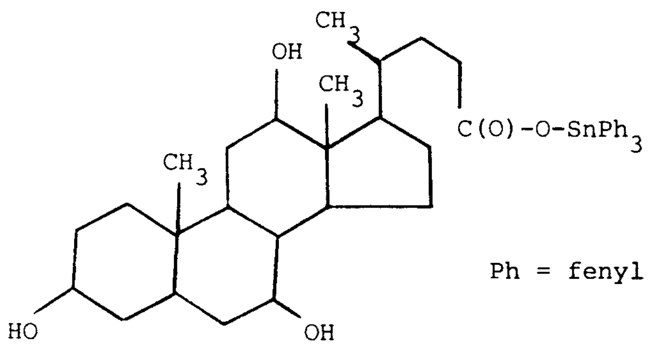

Ph = fenyl

These publications also disclose a number of other tin steroids and their anti-cancer activity, e.g. triphenyl testosteronyl ether, tributyltin deoxycholate, triphenyltin cholesteryl ether, cholesteryl-n-butylstannate, triphenyltin dehydroisoandrosterone, etc..

"Strategy for the Development of Novel Organotin Anti-cancer Agents" by M. Gielen, R. Willem, T. Mancilla, J. Ramharter en E. Joosen from "Silicon, Germanium, Tin and Lead Compounds", Vol. IX, No. 4, 1986, pages 349-365 (Freund Publishing House, London, England) discloses the di-n-butylstannylene derivative of 11-desoxy-17α-hydroxy corticosterone and mentions anti-cancer activity for this compound.

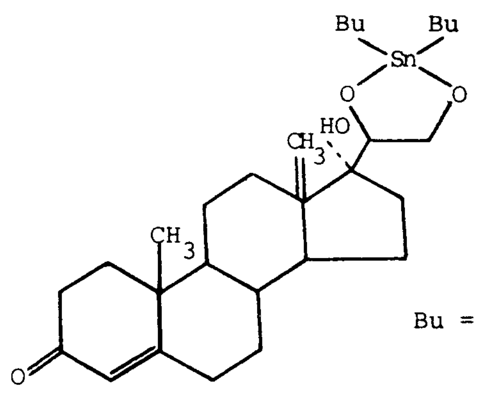

Bu = butyl

This invention provides compositions having excellent anti-tumor activity. These compositions contain as an active ingredient one or more of the following compounds:

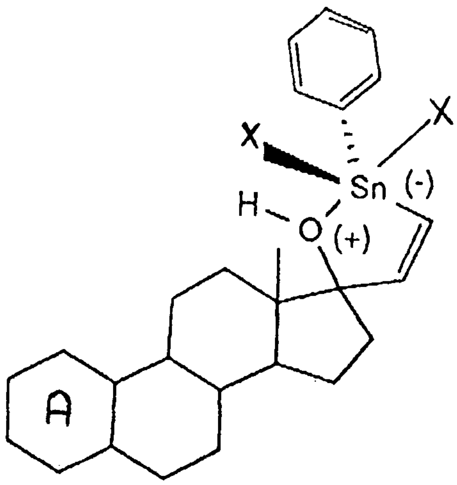

I

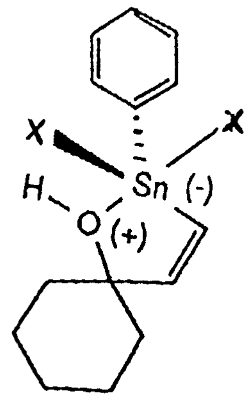

II

wherein

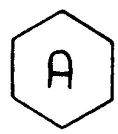

III

represents

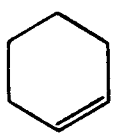

IV

or

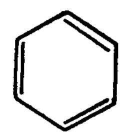

V

which last group may be substituted by a $C_1$-$C_4$ alkoxy group; and X is Cl, Br or I.

Preferred compounds are those of the following formulae:

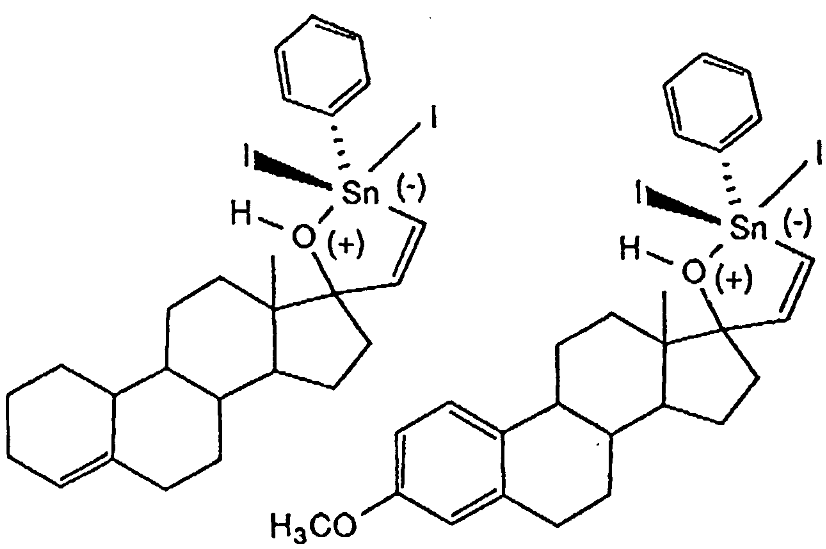

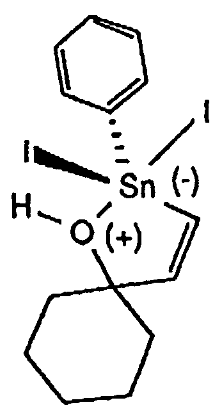

(1) (2) (3)

Compounds (2) and (3) are novel compounds. Compound (1) is known from an article by H. Pan, R. Willem, J. Meunier-Piret and M. Gielen in Organometallics, 1990, 9, pages 2199-2201; however, this article does not mention or suggest the use of this compound as an anti-tumor agent.

The following reaction scheme illustrates the preparation of the above compounds with reference to iodine as the halogenating agent. This type of reaction is disclosed in the above mentioned article from Organometallics 1990, 9, 2199-2201 which discloses the reaction of 17-ethynyl-4-estren-17-ol with triphenyltin hydride, followed by the reaction of the obtained product with one equivalent of iodine and thereafter with a second equivalent of iodine to obtain (Z)-17-[2-(phenyldiiodostannyl) vinyl]-4-estren-17-ol.

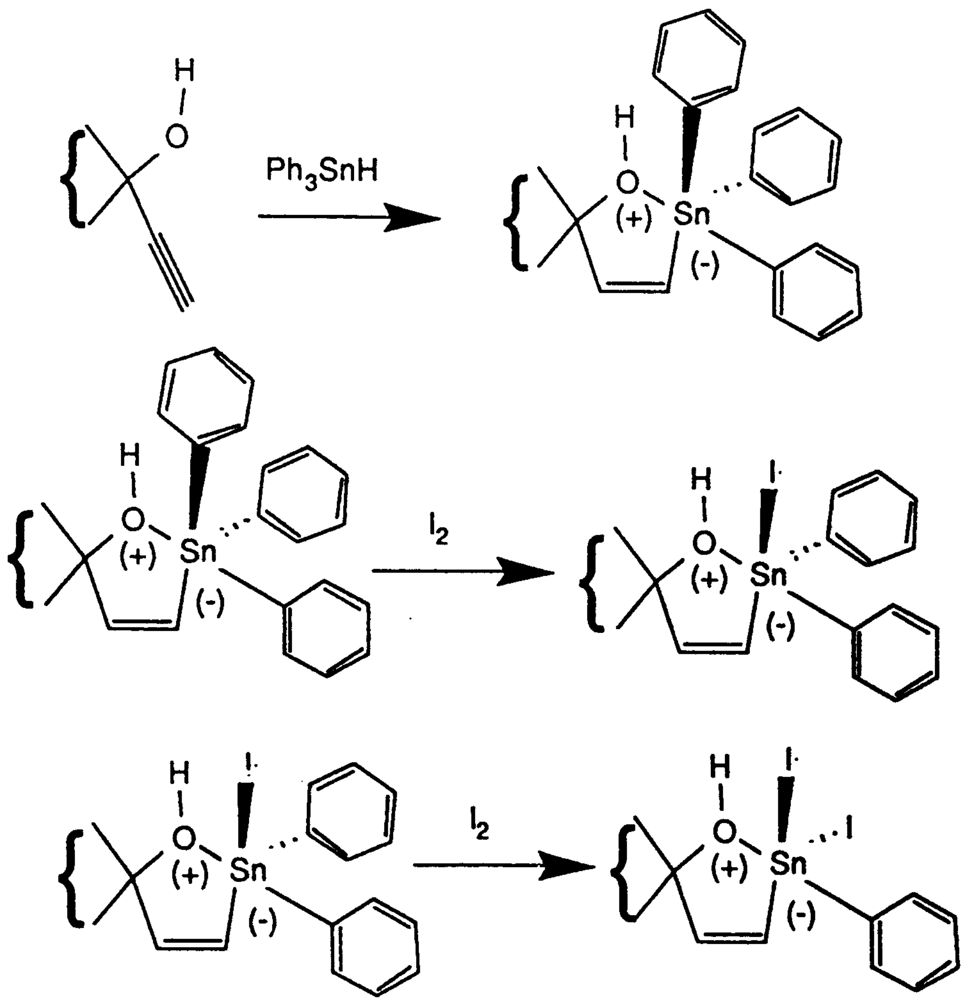


The compounds (1), (2) and (3) as well as their starting compounds, i.e. (4), (5) and (6) respectively,

(4)

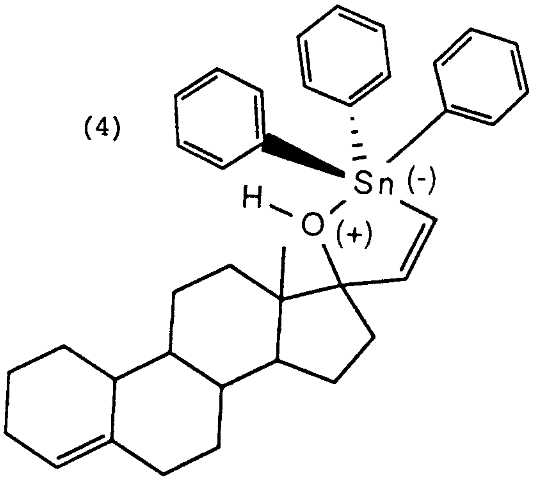

(5)

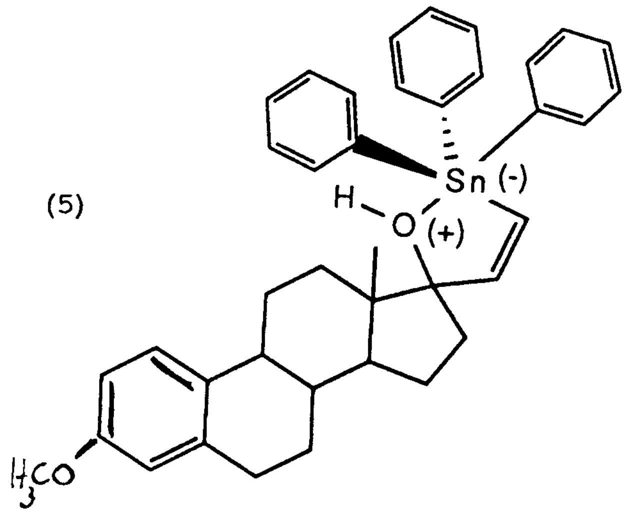

(6)

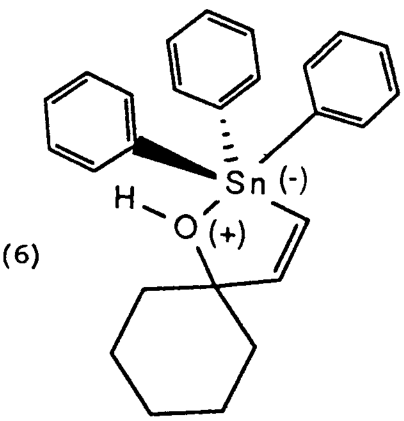

were tested in vitro against the following human tumor cell lines
MCF-7 mammary carcinoma
WiDr colon tumor cells

Also tested was cis-platin, a well known compound for the treatment of human tumors.

The tests were carried out according to the method of R. van Lambalgen and P. Lelieveld, The PIT Method: an automated in vitro technique for drug toxicity testing. Invest. New Drug 5, 161-165 (1987).

The $ID_{50}$ values in ng/ml for the above compounds were determined, i.e. the amount which inhibits 50% of the cell growth.

| $ID_{50}$ values in ng/ml | | |
|---|---|---|
| Compound | MCF-7 | WiDr |
| (1) | 113 | 165 |
| (2) | 49 | 140 |
| (3) | 312 | 1195 |
| (4) | 5943 | 6931 |
| (5) | 2846 | 2361 |
| (6) | 416 | 345 |
| Cis-platin | 850 | 624 |

From the above it can be seen that not only the diiodo compounds show a good anti-tumor activity, but also compound (6), the starting compound for compound (3).

Example 1

1.42 g of 17-ethynyl-4-estren-17-ol (5 mmol) and 1.76 g of triphenyltin hydride (5 mmol) were dissolved in 30 ml of dry ether in a two-necked flask and 10 mg of dibenzoylperoxide was added. After 20 hours at room temperature, the ether was evaporated and the viscous oil was crystallized from ethanol to obtain 2.06 g of white crystals, mp 133-135 °C, yield 65%. 0.25 g of hexaphenylditin (mp 231-232 °C), which dissolves only slightly in ethanol, was obtained as a by-product. After recrystallizing three times from ethanol, white needles were obtained which were studied by X-ray diffraction analysis [elementary analysis: C: 70.4% (calculated for $C_{39}H_{42}O_2Sn$: 70.8%); H: 6.2% (calculated: 6.4%)]. This study showed that the obtained material is indeed compound (4). The resonance at 2.55 ppm of the acetylenic proton of 17-ethynyl-4-estren-17-ol is no longer present in the proton NMR spectrum of (4), but rather, characteristic signals of ethylenic protons are observed, i.e. two doublets at 6.17 and 6.98 ppm, with tin-satellites [$^3J(^1H\text{-}C=C\text{-}^{117/119}Sn)$ = 98/102 and 178/188 Hz, respectively].

Example 2

1.49 g of 17α-ethynylestradiol 3-methyl ether (5 mmol) and 1.76 g of triphenyltin hydride (5 mmol) were dissolved in 30 ml of dry ether in a two-necked flask and 10 mg of dibenzoylperoxide was added. After 20 hours at roomtemperature, the ether was evaporated and the viscous oil was crystallized from ethanol. An amount of 2.2 g of white crystals were obtained, yield 65% [elementary analysis: C: 71.2% (calculated for $C_{38}H_{44}OSn$: 71.8%); H: 7.0% (calculated: 7.0%)]. An amount of 0.2 g of hexaphenylditin was obtained as by-product. The resonance at 2.59 ppm of the acetylenic proton of 17α-ethynylestradiol 3-methyl ether is no longer present in the proton NMR spectrum of compound (5), but rather, characteristic signals of ethylenic protons are observed, i.e. two doublets at 6.21 and 7.06 ppm, with tin-satellites [$^3J(^1H\text{-}C=C\text{-}^{117/119}Sn)$ = 97/100 and 177/187 Hz, respectively]. The aromatic signals of the steroid moiety can be clearly observed as a doublet, a doublet of doublets and another doublet at 6.61, 6.68 and 7.15 ppm, respectively. The methoxy-protons resonate at 3.76 ppm.

Example 3

0.42 g of 1-ethynylcyclohexanol (5 mmol) and 1.76 g triphenyltin hydride (5 mmol) were dissolved in 30 ml of dry ether in a two-necked flask and 10 mg of dibenzoylperoxide was added. After 20 hours at room temperature the ether was evaporated and the viscous oil was crystallized from ethanol, to yield 0.60 g of white crystals, yield: 64%. 0.2 g of hexa-phenylditin was obtained as by-product. The resonance at 2.43 ppm of the acetylenic proton of 1-ethynylcyclohexanol is no longer present in the proton NMR spectrum of compound (6), but rather, characteristic signals of ethylenic protons are observed, i.e. two doublets at 6.34 and 7.00 ppm, with tin-satellites [$^3J(^1H\text{-}C=C\text{-}^{117/119}Sn)$ = 94/98 and 177/185 Hz, respectively].

Example 4

An amount of 50.8 mg of iodine (0.2 mol) dissolved in 5 ml of chloroform was added to 134.8 mg of compound (4) dissolved in 2 ml of chloroform. The colour of iodine disappeared immediately. A proton NMR spectrum of a $CDCl_3$ solution of the obtained compound, in which one of the three phenyl substituents present in compound (4) had been replaced by an I-atom, shows, in addition to the complex parts which is characteristic for the steroid moiety, two vinyl-protons with a slightly changed chemical shift: 6.38 and 6.77 ppm, with tin-satellites [$^3J(^1H\text{-}C=C\text{-}^{117/119}Sn)$ = 126/132 and 240/251 Hz, respectively]. This shows that the obtained compound is indeed Z-17-(2-diphenyliodostannylvinyl)-4-estren-17-ol, compound (7).

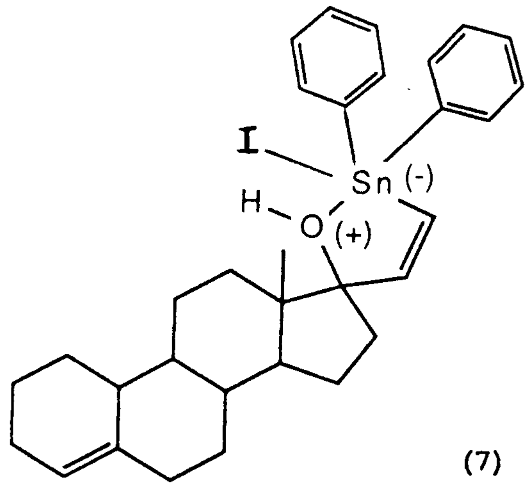

(7)

Example 5

An amount of 50,8 g of iodine (0.2 mol) dissolved in 5 ml of chloroform, were added to 141 mg of compound (5) dissolved in 2 ml of chloroform. The colour of the iodine disappears immediately. A proton-NMR spectrum of a $CDCl_3$ solution of the obtained compound, in which one of the three phenyl substituents, which where present in compound (5), had been replaced by an I-atom, shows, in addition to the complex parts which is characteristic for the steroid moiety, two vinyl-protons having a slightly changed chemical shift: 6.44 and 6.86 ppm, with tin-satellites [$^3J(^1H\text{-}C=C\text{-}^{117/119}Sn)$ = 123/130 and 236/248 Hz, respectively].

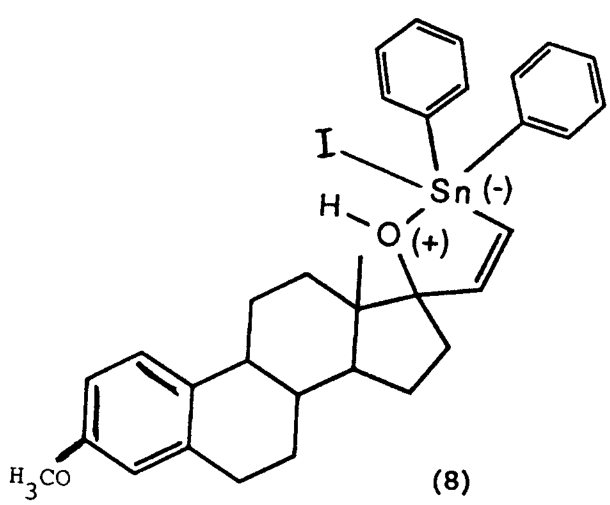

(8)

Example 6

After the addition of 50.8 mg of iodine, dissolved in 5 ml of ethanol, to the $CDCl_3$ solution of compound (7) the colour disappears much slower. The proton-NMR spectrum of the obtained compound in $CDCl_3$ again shows the complicated steroid part and two doublets at 6.40 and 6.01 ppm, with tin-satellites [$^3J(^1H\text{-}C=C\text{-}^{117/119}Sn)$ = 195-204 and 306-317 Hz, respectively], which proves that this compound is indeed Z-17-(2-phenyldiiodostannyl)vinyl]-4-estren-17-ol.

Example 7

After the addition of 50.8 mg of iodine, dissolved in 5 ml of ethanol, to the $CDCl_3$ solution of (8), the colour disappears much slower. The proton NMR spectrum of the obtained compound in $CDCl_3$ again shows the complicated steroid part and one of the two expected doublets at 6.48 ppm. The other doublet at 6.72 ppm is situated in the complex region of the aromatic protons of the steroid moiety.

Example 8

101.6 mg of iodine (0.4 mol) dissolved in 5 ml of chloroform, was added to 95 mg (0.2 mol) of (6) dissolved in 2 ml of chloroform. After the disappearence of the colour of iodine, the solvent is evaporated under reduced pressure. A proton NMR spectrum of a $CDCl_3$ solution of the obtained compound, in which two of the three phenyl substituents present in compound (6) had been replaced by two I-atoms, shows, in addition to the signals of the cyclohexane part, doublets at 6.47 and 6.57 ppm, with tin-satellites $[^3J(^1H\text{-}C{=}C\text{-}^{117/119}Sn) = 188/197$ and 311/329, respectively]. This proves that the obtained compound is indeed 1-(2-phenyldiiodostannyl)vinyl]cyclohexanol, compound (3).

**Claims**

**1.** Anti-tumor compositions, containing as an active ingredient one or more of the following compounds:

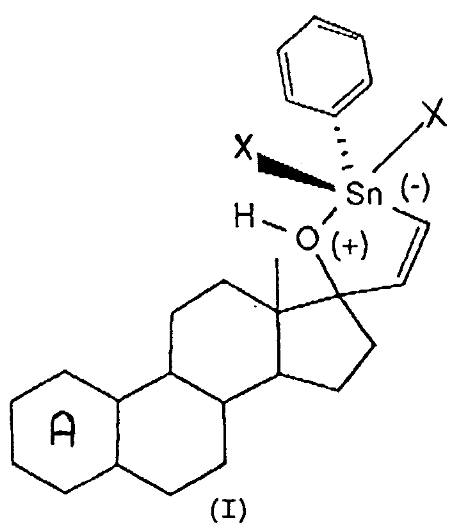

(I)

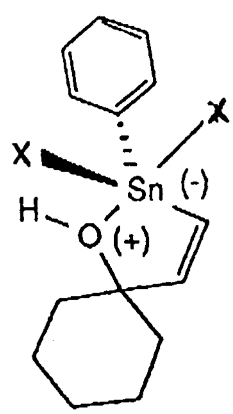

(II)

wherein

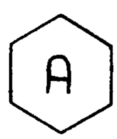

(III)

represents

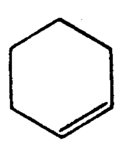

(IV)

or

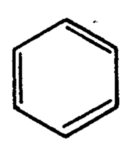

(V)

which last group may be substituted by a $C_1$-$C_4$ alkoxy group;
and X is Cl, Br or I.

**2.** Anti-tumor composition according to claim 1, containing a compound having the formula (1):

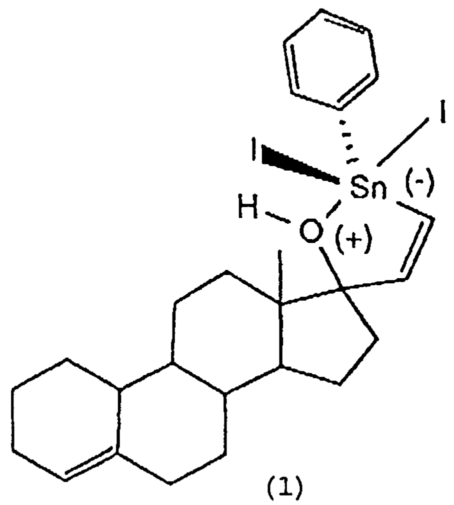

(1)

3. Anti-tumor composition according to claim 1, containing a compound having the formula (2)

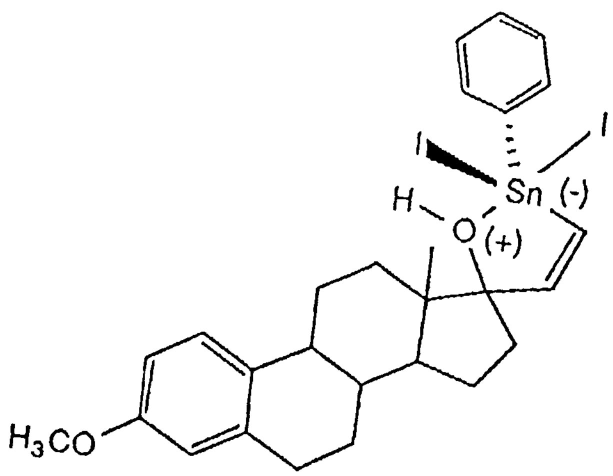

(2)

4. Anti-tumor composition according to claim 1, containing a compound having the formula (3)

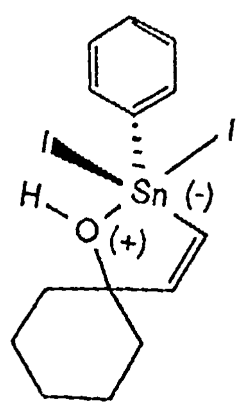

(3)

**5.** A compound having the formula (2):

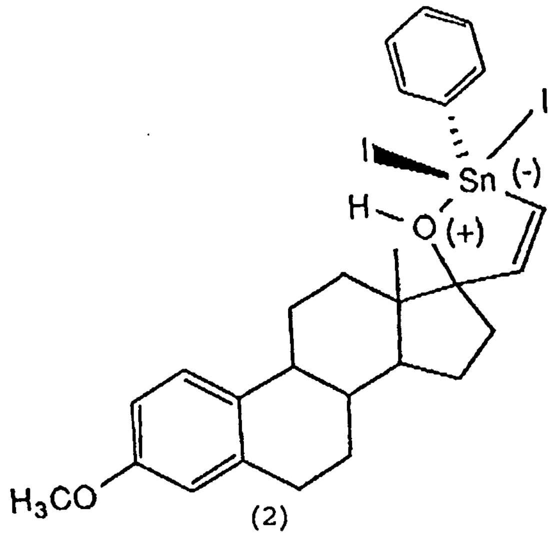

(2)

**6.** A compound having the formula (3):

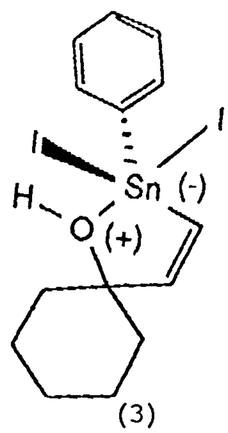

(3)

**7.** A compound having the formula (6):

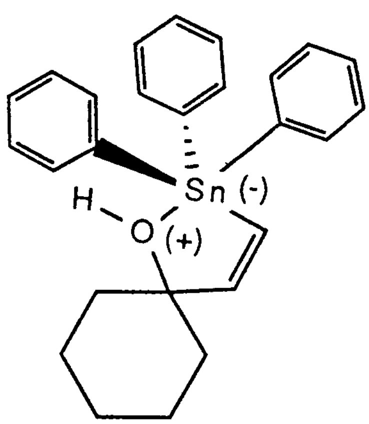

(6)

**8.** Anti-tumor composition, containing as an active ingredient a compound having the formula (6):

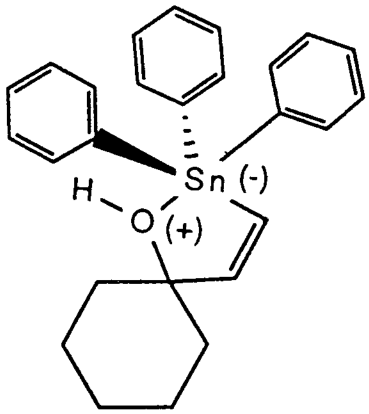

(6)

**Claims for the following Contracting States : ES, GR**

**1.** A process for preparing anti-tumor compositions, **characterized** by combining as an active ingredient one or more of the following compounds:

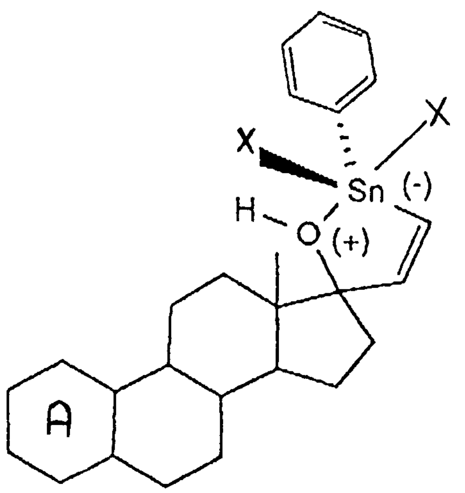

(I)

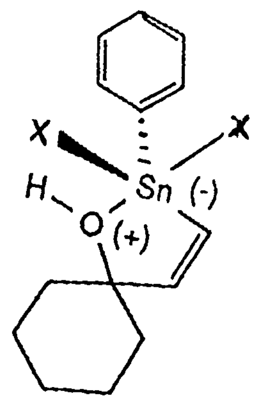

(II)

wherein

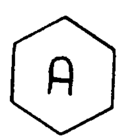

(III)

represents

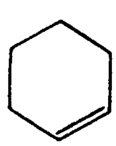

(IV)

or

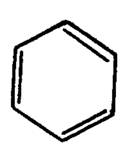

(V)

which last group may be substituted by a $C_1$-$C_4$ alkoxy group;
and X is Cl, Br or I;
with a carrier or diluent for said active ingredient.

2. A process according to claim 1, **characterized** by using as said active ingredient a compound of formula (1):

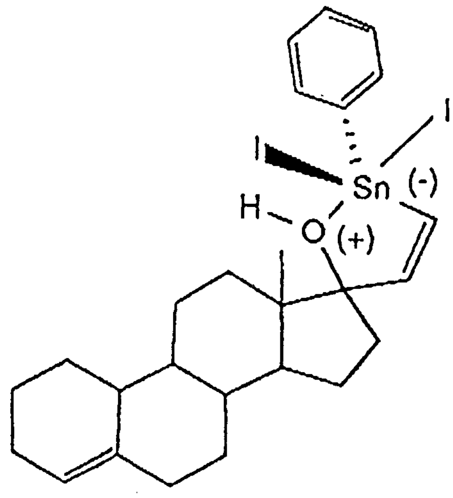

(1)

3. A process according to claim 1, **characterized** by using as said active ingredient a compound having the formula (2):

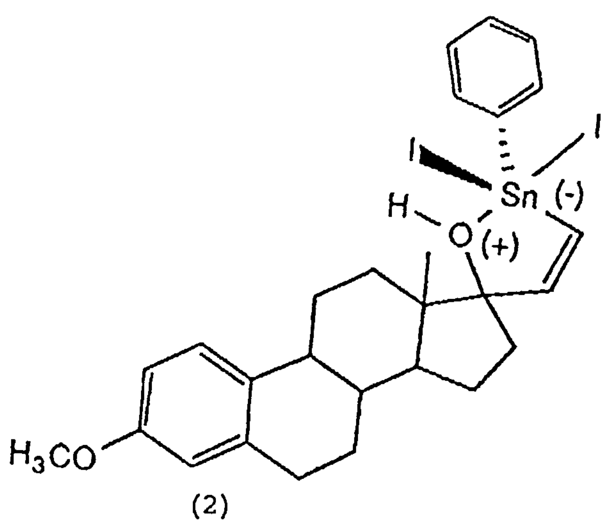

(2)

**4.** A process according to claim 1, **characterized** by using as said active ingredient a compound having the formule (3):

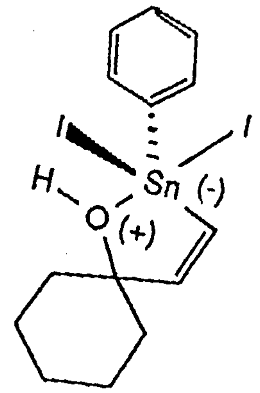

(3)

**5.** A process for preparing anti-tumor compositions, **characterized** by combining as an active ingredient a compound having the formula (6):

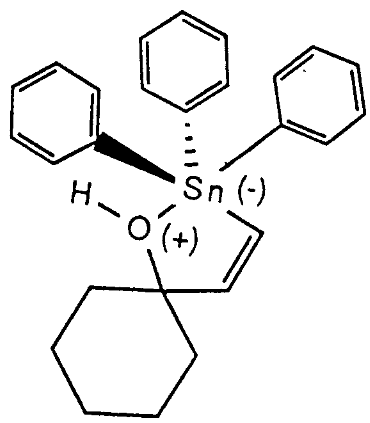

(6)

with a carrier or diluent for said active ingredient.

European Patent Office

# EUROPEAN SEARCH REPORT

Application Number

EP 90 20 2936

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | Ed.: N. CARDARELLI: "Tin as a vital nutrient", chapter 15, 1985, pages 189-195, CRC Press, US; S.V. KANAKKANATT: "Synthesis of tin steroids and the relation between structure and anticancer activity"<br>* Page 190, 1st - 3rd paragraph; page 192, last paragraph: "Conclusion and Discussion" - end of article *<br>--- | 1,2 | A 61 K 31/555<br>C 07 F 7/22 |
| Y | THE AUSTRALIAN JOURNAL OF EXPERIMENTAL BIOLOGY AND MEDICAL SCIENCE, vol. 62 (part 2), April 1984, pges 199-208, The University of Adelaide, AU; N.F. CARDARELLI et al.: "Organotin implications in anticarcinogenesis. Background and thymus involvement"<br>* Page 199, summary; page 200, last paragraph - page 201, line 22; page 206, 2nd paragraph (below tables) - end *<br>--- | 1,2 | |
| | | | TECHNICAL FIELDS SEARCHED (Int. Cl.5) |
| D,Y | ORGANOMETALLICS, vol. 9, 1990, pages 2199-2201; H. PAN et al.: "Intramolecular O-Sn coordination in (Z-17-[2-(triphenylstannyl(vinyl]-4-estren-17-ol: Evidence by X-ray-diffraction analysis and iodo demetalation"<br>* Page 2199, figure I, compound 3 *<br>--- | 1,2 | A 61 K<br>C 07 F |
| Y | US-A-4 634 693 (N.F. CARDARELLI et al.)<br>* Abstract; column 1, lines 26-42; columns 15/16, tables IV and V; claims 1-7 *<br>--- -/- | 1,2 | |

~~The present search report has been drawn up for all claims~~

| Place of search | Date of completion of the search | Examiner |
|---|---|---|
| THE HAGUE | 04-07-1991 | KRAUTBAUER B. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

## CLAIMS INCURRING FEES

The present European patent application comprised at the time of filing more than ten claims.

☐ All claims fees have been paid within the prescribed time limit. The present European search report has been drawn up for all claims.

☐ Only part of the claims fees have been paid within the prescribed time limit. The present European search report has been drawn up for the first ten claims and for those claims for which claims fees have been paid,

namely claims:

☐ No claims fees have been paid within the prescribed time limit. The present European search report has been drawn up for the first ten claims.

## LACK OF UNITY OF INVENTION

The Search Division considers that the present European patent application does not comply with the requirement of unity of invention and relates to several inventions or groups of inventions,
namely:

See sheet -B-

☐ All further search fees have been paid within the fixed time limit. The present European search report has been drawn up for all claims.

☐ Only part of the further search fees have been paid within the fixed time limit. The present European search report has been drawn up for those parts of the European patent application which relate to the inventions in respect of which search fees have been paid,

namely claims:

☒ None of the further search fees has been paid within the fixed time limit. The present European search report has been drawn up for those parts of the European patent application which relate to the invention first mentioned in the claims,

namely claims: 2 and 1(partially)

European Patent Office

# EUROPEAN SEARCH REPORT

Application Number

EP 90 20 2936

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-4 547 320 (E.J. BULTEN et al.) * Abstract; column 1 - column 2, line 47; claims 1-7 * --- | 1,2 | |
| D,Y | SILICON, GERMANIUM, TIN AND LEAD COMPOUNDS, vol. 9, no. 4, 1986, pages 349-365; M. GIELEN et al.: "Strategy for the development of novel organotin anti-cancer agents" * Whole article, especially page 159, last paragraph - page 361, 2nd paragraph * --- | 1,2 | |
| Y | JOURNAL OF PHARMACOBIODYNAMICS, vol. 10, no. 1, January 1987, page S-2, Pharmaceutical Society of Japan; Y. ARAKAWA et al.: "Anti-tumor activity and anti-imflammatory action of organotin compounds" * Left-hand column - right-hand column, line 5 * ----- | 1,2 | |
| | | | TECHNICAL FIELDS SEARCHED (Int. Cl.5) |

~~The present search report has been drawn up for all claims~~

| Place of search | Date of completion of the search | Examiner |
|---|---|---|
| THE HAGUE | 04-07-1991 | KRAUTBAUER B. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

## LACK OF UNITY OF INVENTION

The Search Division considers that the present European patent application does not comply with the requirement of unity of invention and relates to several inventions or groups of inventions,
namely:

1. Claims 2 and 1(partially): Anti-tumor composition containing a compound having the formula (1) (corresponding to (I) wherein A (III) represents (IV)); use of a known.

2. Claims 3-8 and 1(partially): New compounds having formula (2),(3),(6) and their use as anti-tumor compositions.